# FASCICULE DE BREVET EUROPEEN

(11) **EP 1 708 740 B1**
(45) Date de publication et mention de la délivrance du brevet: **29.09.2010**
(21) Numéro de dépôt: 05717439.3
(22) Date de dépôt: 18.01.2005
(51) Int. Cl.: A61K 38/55, A61P 1/00

(54) **INHIBITEURS DE PROTÉASES POUR LE TRAITEMENT DE PATHOLOGIES DIGESTIVES**
PROTEASEHEMMER ZUR BEHANDLUNG VON VERDAUUNGSSTÖRUNGEN
PROTEASE INHIBITORS FOR THE TREATMENT OF DIGESTIVE PATHOLOGIES

(30) Priorité: 19.01.2004 FR 0400446
(43) Date de publication de la demande: 11.10.2006
(73) Titulaire: Rytek, 34160 Saint Genies des Mourgues (FR)
(72) Inventeur: BUENO, Lionel, F-31840 Aussonne (FR)
(74) Mandataire: Tezier Herman, Béatrice
(86) Numéro de dépôt international: PCT/FR2005/000108
(87) Numéro de publication internationale: WO 2005/077406

(56) Documents cités:
- EP-A- 0 958 833
- WO-A-99/55323
- WO-A-02/070468
- WO-A-03/077893
- US-A1- 2003 138 423

## Description

La présente demande concerne des compositions et méthodes pour le traitement de pathologies intestinales. Elle concerne également des compositions et méthodes permettant de réguler la perméabilité paracellulaire de l'épithélium intestinal. Les compositions et méthodes de l'invention reposent notamment sur l'utilisation d'inhibiteurs de protéases modulant l'ouverture des jonctions serrées de l'épithélium intestinal. L'invention est utilisable pour le traitement préventif ou curatif de pathologies variées, telles que les troubles fonctionnels digestifs (TFD), plus particulièrement les troubles fonctionnels intestinaux (TFI), et notamment le syndrome de l'intestin irritable (SII encore appelé « Irritable Bowel Syndrome », IBS), les hyperalgésies et autres douleurs abdominales, etc., chez les mammifères, notamment les humains.

L'épithélium intestinal est le centre d'échanges très importants entre le milieu extérieur et l'organisme. Ces échanges peuvent s'effectuer soit à travers les cellules de l'épithélium, soit par des réseaux parallèles. Ainsi, le transport d'eau ou d'électrolytes, ou encore l'absorption de petites molécules (poids moléculaire généralement inférieur à 1000 Da environ) au niveau de la muqueuse gastrique, intestinale ou colique, s'effectue par voie transcellulaire, à travers les cellules épithéliales ou entérocytes. Par contre, l'absorption de grosses molécules et le passage d'antigènes, de toxines ou de cellules immunitaires se fait principalement par voie paracellulaire, au niveau de «jonctions serrées », qui sont disposées entre les cellules épithéliales.

Les jonctions serrées (« JS ») épithéliales (ou *« tight junction* », « TJ ») sont des structures de liaison entre les cellules bordant les épithéliums muqueux (tube digestif, poumons). Ces structures assurent et contrôlent le transport transépithélial paracellulaire, de l'extérieur vers la sous-muqueuse, de macromolécules variées (allergènes, irritants, toxines, microorganismes). Ces structures permettent également la migration de cellules immunitaires (e.g., immunocytes) vers l'extérieur (tube digestif). Les jonctions serrées sont des structures souples constituées d'un assemblage complexe de protéines transmembranaires (occludines, claudines) et de protéines cytoplasmiques (protéines zona ocludens ZO-1, ZO-2, ZO-3, protéines AF7, cinguline ou 7H6, etc.), qui sont associées à des éléments du cytosquelette (filaments de myosine, d'actine, etc.).

Parmi les troubles fonctionnels digestifs (TFD), on distingue les douleurs abdominales sans trouble de l'élimination et les troubles fonctionnels intestinaux. Les douleurs abdominales sans trouble de l'élimination peuvent être provoquées par des allergies ou des intolérances alimentaires ou se manifester dans le cadre de la maladie coeliaque par exemple. Les troubles fonctionnels intestinaux affectent 15 à 20% de la population et se traduisent par des symptômes dyspeptiques et/ou intestinaux pour lesquels aucune cause organique n'a jusqu'ici été identifiée et qui nécessitent un traitement spécifique. La caractéristique commune des troubles fonctionnels digestifs répondant aux critères de ROME est la douleur abdominale, présente en période postprandiale, qu'elle soit localisée en partie haute (dyspepsie) ou en partie basse de l'abdomen. Un trouble particulier qui affecte la partie basse de l'abdomen est le Syndrome de l'Intestin Irritable (SII). La douleur des troubles fonctionnels intestinaux est le plus souvent soulagée par la défécation.

La physiopathologie des troubles fonctionnels digestifs (TFD) est mal connue mais les récentes investigations cliniques montrent l'existence chez ces patients d'un abaissement du seuil de perception douloureuse à la distension (hyperalgésie viscérale) traduisant un état d'hypersensibilité digestive. Celui-ci semble relever en premier lieu d'une sensibilisation de mécanorécepteurs pariétaux par des médiateurs pro-inflammatoires. En effet l'existence de modifications de structure ou de densité de certaines cellules du système immunitaire au niveau de la sous-muqueuse en particulier du colon plaide en faveur d'une altération de l'équilibre immunitaire avec la microflore résidente. Une densité accrue de mastocytes ou de cellules entérochromaffines a été détectée dans des biopsies iléales et coliques chez des sujets atteints de SII. Cet état micro-inflammatoire digestif favorise la sensibilisation des terminaisons des neurones afférents primaires et cette sensibilisation pourrait conduire à une facilitation secondaire de la transmission, vers le cerveau, des messages à caractère nociceptif. Les études d'imagerie fonctionnelle indiquent en effet des modifications caractéristiques des aires de projection corticale de ces messages nerveux chez les sujets atteints de TFD.

Un tel processus de sensibilisation de l'intestin à la douleur peut être induit par des facteurs tels que le stress, des pathogènes, des allergènes, des enzymes de type trypsine ou tryptase par exemple, des sels biliaires, des xénobiotiques, des molécules chimiques de type glycérol, TNBS ou taurocholate par exemple et/ou des séquelles résultant d'une infection ou d'une opération chirurgicale. Toutefois, les mécanismes par lesquels ces facteurs sont capables d'initier le phénomène de sensibilisation ne sont pas clairement documentés *in vivo.* Lorsque des allergènes, des pathogènes et/ou des molécules chimiques par exemple sont absorbés, ils entrent en contact avec la paroi épithéliale intestinale qui empêche leur introduction dans l'organisme et leur mise en contact avec les cellules de l'immunité. Toutefois, pour qu'une sensibilité se développe, cela implique que certains allergènes, pathogènes et/ou molécule chimique sont capables de traverser cet épithélium pour interagir avec les cellules immunitaires. Les conditions dans lesquelles ce transfert est possible demeurent peu documentées *in vivo.* Ainsi, bien que certains rapports suggèrent, sur des cultures de cellules *in vitro,* un rôle des jonctions serrées dans ce processus, aucune démonstration n'a été apportée de l'implication de ces jonctions *in vivo* dans le développement d'une sensibilisation. De même, si Coremans et al. (Ital J Gastroenterol 1991 (8) S 1 :39-40) suggèrent un lien entre les sels biliaires, perfusés par voie intra-colique, et les douleurs abdominales d'un sujet présentant un intestin irritable, aucun résultat présenté n'établit de corrélation entre les jonctions serrées et un processus de sensibilisation à la douleur de l'épithélium intestinal.

Si la malabsorption des sels biliaires n'est observée que chez une faible proportion des sujets présentant un intestin irritable, des altérations de la perméabilité trans-epithéliale sont décrites chez les sujets développant un SII après gastro-entérite (SPILLER et al. Gut 2000 ; 47 : 804-11) mais sans qu'il soit fait mention de modifications de la perméabilité paracellulaire laquelle n'est pas étudiée.

Une corrélation entre le syndrome de l'intestin irritable et la présence accrue de microorganismes au niveau de la sous-muqueuse a été mise en évidence au travers d'études expérimentales (AGA. 1999), de même qu'un lien a pu être constaté entre les maladies inflammatoire chroniques de l'intestin ou les troubles fonctionnels intestinaux et des modifications du degré d'activation immunitaire (inflammation) de la paroi intestinale. Cependant, ces observations préliminaires n'ont pas été confirmées ou n'ont pas donné lieu à de nouvelles approches thérapeutiques.

La présente invention résulte de la mise en évidence de l'activation, par les protéases bactériennes libérées dans la lumière colique, des récepteurs PAR (proteinase-activated receptors), situés sur les membranes des cellules épithéliales, qui modulent l'ouverture des jonctions serrées, cette ouverture pouvant entraîner un état d'hyperalgésie.

L'origine bactérienne des protéases mentionnées ci-dessus a été démontrée par le fait qu'un traitement oral de 10 jours par un cocktail d'antibiotiques détruisant la flore réduit la perméabilité des jonctions serrées, celle-ci étant restaurée par la perfusion intracolique d'un surnageant de contenu colique normal. Les inventeurs ont par ailleurs démontré qu'un cocktail d'inhibiteurs de protéases perfusé dans la lumière colique réduisait la perméabilité paracellulaire colique et la sensibilité viscérale à la distension.

Le rôle *in vivo* des jonctions serrées de l'épithélium intestinal dans le processus de sensibilisation à la douleur a par ailleurs été mis en évidence dans la demande de brevet WO 03/077893. L'"ouverture" des jonctions serrées de l'épithélium du colon par différentes molécules ou par le stress entraîne une hyperalgésie spontanée ou une hypersensibilité à la distension (état caractéristique de l'IBS). Les résultats obtenus dans le cadre de cette invention ont établi pour la première fois et de façon surprenante que l'ouverture des jonctions serrées provoque un état d'hyperalgésie retardé durable.

La présente invention découle de la mise au point de nouvelles stratégies thérapeutiques pour le traitement de pathologies intestinales, basées sur une modulation de la perméabilité para-cellulaire de l'épithélium intestinal à l'aide d'inhibiteurs de protéases. En particulier, la présente invention propose une approche thérapeutique des pathologies intestinales basée sur l'emploi d'inhibiteurs de protéases permettant de contrôler l'ouverture des jonctions serrées de l'épithélium intestinal. Ainsi, ces inhibiteurs de protéases permettent de moduler la tension du cytosquelette des cellules épithéliales intestinales ou de réguler directement, de préférence diminuer, voire bloquer, l'ouverture des jonctions serrées de l'épithélium intestinal. Cette approche permet donc de contrôler l'ouverture et la fermeture des jonctions serrées de l'épithélium intestinal, sans nécessairement recourir à une synthèse protéique *de novo* et/ou à des dégradations protéiques et/ou structurales importantes au niveau de l'épithélium. Cette stratégie permet de réguler la perméabilité de l'épithélium intestinal de manière spécifique, fine et réactive, et ainsi d'agir sur le transfert d'allergènes, de pathogènes et/ou de molécules chimiques vers les cellules de l'immunité. Cette stratégie est particulièrement adaptée à l'obtention d'un effet biologique rapide et contrôlable dans le temps (réversible).

A cet égard, les résultats présentés ci-dessous montrent qu'une substance susceptible de relâcher les jonctions serrées épithéliales (peptides activateurs du récepteur PAR-2 tels que le peptide SLIGRL) déclenche une hyperalgésie retard, une hypersensibilité à la distension et une augmentation de la perméabilité colique. Les résultats présentés dans les exemples montrent également que la suppression de cette augmentation de la perméabilité paracellulaire par un inhibiteur de protéases ou un mélange (cocktail) d'inhibiteurs de protéases supprime ou réduit cette hyperalgésie caractéristique des TFD et en particulier des SII.

Un premier objet de l'invention réside donc plus particulièrement dans l'utilisation d'au moins un inhibiteur de protéases, pour la préparation d'un médicament destiné au traitement préventif ou curatif des troubles fonctionnels digestifs. L'invention concerne également une telle utilisation pour la préparation d'un médicament destiné au traitement préventif ou curatif de l'hyperalgésie survenant dans le cadre de pathologies intestinales. L'inhibiteur de protéase est de préférence un inhibiteur de protéases intracoliques.

Un autre objet de l'invention réside dans une méthode de traitement préventif ou curatif de pathologies intestinales caractérisées par un état d'hyperalgésie, comprenant l'administration à un sujet d'une quantité efficace d'au moins un inhibiteur de protéases.

Les inhibiteurs de protéases semblent agir en contrôlant l'ouverture des jonctions serrées de l'épithélium intestinal. Ces inhibiteurs sont en particulier des inhibiteurs qui modulent la tension du cytosquelette de cellules épithéliales intestinales ou des inhibiteurs qui diminuent, voire bloquent, l'ouverture des jonctions serrées de l'épithélium intestinal.

L'invention repose ainsi sur l'utilisation d'inhibiteurs de protéases modulant la tension et l'état de contraction du cytosquelette des cellules de l'épithélium intestinal ou empêchant une ouverture trop importante des jonctions serrées qui aboutit à une hyperalgésie ou à une hypersensibilité à la distension de l'intestin.

Les protéines composant les jonctions serrées sont associées au cytosquelette des cellules qu'elles relient. Il est proposé dans le cadre de l'invention que la tension du cytosquelette ou l'ouverture des jonctions serrées puisse être modulée chez des sujets atteints de maladies ou désordres intestinaux pour agir de manière non destructrice et transitoire sur la perméabilité de leur épithélium intestinal. Ainsi, la contraction du cytosquelette doit favoriser l'ouverture des jonctions serrées, tandis qu'un relâchement du cytosquelette (ou qu'une inhibition de la contraction) doit favoriser la fermeture des jonctions. Il est possible également de moduler directement les jonctions serrées, en particulier sur les protéines les constituant, en diminuant ou en bloquant leur ouverture.

On utilise donc préférentiellement dans le cadre de l'invention des inhibiteurs de protéases qui modulent la contraction du cytosquelette de cellules épithéliales intestinales (notamment humaines) ou qui contrôlent l'ouverture des jonctions serrées de l'épithélium intestinal (notamment humain). Selon la condition à traiter, on utilise des inhibiteurs de protéases qui inhibent la contraction du cytosquelette de cellules épithéliales intestinales, ou qui activent ou favorisent celle-ci

Un inhibiteur de protéase est considéré comme modulant la tension du cytosquelette lorsqu'il module l'ouverture des jonctions serrées. Un effet inhibiteur de la contraction ou de la tension des filaments d'actine et/ou de myosine ne doit pas nécessairement être complet ou total, mais il suffit qu'il diminue la contraction ou la tension du cytosquelette suffisamment pour réduire l'ouverture des jonctions serrées.

La réduction de l'ouverture des jonctions serrées correspond préférentiellement à une diminution minimale d'environ 25%, avantageusement d'environ 30%, de façon encore plus préférée d'environ 50% de la perméabilité paracellulaire de l'épithélium intestinal. La perméabilité paracellulaire peut être mesurée à l'aide d'un marqueur tel que le ⁵¹Cr-EDTA lequel après son passage dans le sang est mesuré dans les urines pendant 24 heures (cf. : exemple 1).

Les inhibiteurs de protéases utilisés sont de préférence les inhibiteurs agissant sur les sérine-protéases et/ou les métalloprotéases. De tels inhibiteurs sont particulièrement actifs pour réduire l'action des protéases bactériennes sur la perméabilité colique.

Les inhibiteurs de protéases utilisés sont avantageusement des molécules, qui peuvent être sous forme isolée ou sous forme de cocktail, de combinaison, d'extraits biologiques, etc. Ces molécules peuvent être synthétiques, semi-synthétiques ou biologiques, notamment d'origine animale, virale, végétale ou bactérienne.

On peut notamment citer comme inhibiteurs de proteases, les inhibiteurs sélectifs ou non sélectifs de sérine-protéases [serpine et ses dérivés, Aprotinine, N-tosyl-L-phenylalanyl chlorométhyle cétone (TPCK), dichloroisocoumarine, nexin-1, AEBSF-HCl, Antipain, benzamidine, Leupeptine, TLCK, Ovomucoide, fluorure de phénylméthyle sulfonyle (PMSF), et les extraits de soja] et de métallo-protéases (Amastatine, Arphamenine, Bestatin, Diprotin A, Phosphoramidon) ainsi que les molécules non-spécifiques utilisées comme antiviraux (amprenavir, indinavir, lopinavir, ritonavir, saquinavir, nelfinavir et atazanavir).

Les inhibiteurs de protéases peuvent être utilisés seuls ou en combinaisons et/ou en association avec d'autres agents actifs, comme par exemple d'autres substances actives utilisées dans le traitement du syndrome de l'intestin irritable.

Les inhibiteurs de protéases sont ainsi éventuellement utilisés en combinaison et/ou en association avec des composés qui diminuent ou bloquent l'ouverture des jonctions serrées de l'épithélium intestinal, notamment par modulation de la tension du cytosquelette, ou qui augmentent l'ouverture de celles-ci.

L'activité de ces composés peut être directe ou indirecte, c' est-à-dire dirigée sur les constituants mêmes du cytosquelette ou sur des régulateurs de sa tension. Bien que non limitatif, on préfère les composés agissant de manière directe sur la tension du cytosquelette. En outre, on préfère également des composés présentant une activité sélective sur la tension du cytosquelette, c'est-à-dire typiquement des composés qui n'affectent pas directement la structure des protéines constitutives des jonctions serrées. De même, l'inhibiteur de protéases selon l'invention, n'affecte de préférence pas directement la structure des protéines constitutives des jonctions serrées.

Différents types de composés peuvent être utilisés en combinaison avec des inhibiteurs de protéases dans le cadre de la présente demande. Ainsi, au sens de l'invention, le terme « composé » doit être pris dans un sens large, c'est-à-dire comme désignant tout agent, substance, composition, condition, traitement ou procédé permettant de moduler l'ouverture des jonctions serrées de l'épithélium intestinal. Il s'agit avantageusement d'un agent (e.g., d'une molécule) ou d'une combinaison ou association d'agents. Des exemples de tels composés sont indiqués dans la demande de brevet international n° WO 03/077893. Il s'agit notamment d'inhibiteurs de la kinase de la chaîne légère de la myosine (MLCK). Un exemple particulier d'inhibiteurs sélectif de MLCK est le composé ML-7 {1-(5-iodonaphtalène-1-sulfonyl)-1H-hexahydro-1,4-diazepine} (Makishima M. et al. FEBS Lett. 1991; 287:175). D'autres exemples de tels inhibiteurs sont notamment le composé ML-9 (Wilson DP.et al. J Biol Chem. 2001;13: 165) ou d'autres non sélectifs: Wortmannin (Warashina A. Life Sci 2000; 13: 2587-93), H-7 (Piao Zf et al. Mol Cell Biol Res Commun 2001 ;4: 307-12) et KT 7692 (Warashina A. Life Sci 2000; 13: 2587-93). D'autres cibles agissant sur la tension du cytosquelette de composés utilisables en combinaison avec des inhibiteurs de protéases sont notamment les protéines de liaison à la myosine, telles que par exemple la cinguline, ou les molécules de jonction, telles que la cadherine-E, la catenine-α ou les desmosomes. La modulation de l'activité ou de l'expression de ces protéines permet de réguler la tension du cytosquelette, dans le cadre de la présente invention. On peut également utiliser des inhibiteurs de protéases combinés à des composés inhibiteurs de la synthèse de protéines ou autres molécules assurant la liaison entre les protéines du cytosquelette et les protéines des jonctions serrées. Il est également possible d'utiliser dans le cadre de l'invention des inhibiteurs de kinases activées par les mitogènes (MAPKK), notamment de la kinase MEK ou de la kinase-PI3, tels que les composés PD098,059 {2-(Amino-3-methoxyphenyl)-4H-1-benzopyran-4-one}(Alessi et al. J.Biol.Chem.1995; 270, 27589) ou LY294002 {2-(4-Morpholinyl)-8-phenyl-1(4H)-benzopyran-4-one} (Vlahos et al.J.Biol.Chem 1994;269: 5241). D'autres molécules utilisables pour réguler, de manière indirecte, la tension du cytosquelette, sont des facteurs de croissance, tels que le facteur de croissance hépatique (HGF), le facteur de croissance endothélial (EGF) ou certaines cytokines susceptibles d'être libérées par les immunocytes, telles que les interleukines-1, -4, -13, ou les facteurs tels que IGF-1 ou l'interféron gamma. Une autre approche permettant de réguler de manière indirecte la tension du cytosquelette repose sur l'utilisation du peptide GLP2 (« glucagon-like peptide 2 ») ou de ses dérivés, qui peuvent modifier la perméabilité de l'épithélium intestinal par un effet indirect sur la contraction du cytosquelette. De même, certaines molécules agissant sur des récepteurs situés au pôle apical des cellules épithéliales (ex: récepteurs aux protéinases; PAR-2) peuvent agir indirectement sur le cytosquelette. D'autres agents actifs sont par exemple les composés anti-cholinergiques, les prokinétiques, les anti-diarrhéiques, les modificateurs de la motricité digestive, etc. Ces différents agents peuvent être utilisés en combinaison thérapeutique, et administrés sous forme séparée, combinée, étalée dans le temps ou concomitante.

Un autre objet de l'invention réside ainsi dans un produit, un cocktail ou une association pharmaceutique comprenant au moins un inhibiteur de protéases et au moins un autre agent actif sélectionné parmi les composés anti-cholinergiques, les substances prokinétiques, les anti-diarrhéiques, les laxatifs ou les modificateurs de la motricité, de la viscéro-sensibilité (ou de la sensibilité digestive), en vue d'une utilisation combinée, séparée ou espacée dans le temps.

La présente invention peut être utilisée pour le traitement ou la prise en charge de pathologies ou désordres du système digestif caractérisés par un état d'hyperalgésie, notamment de troubles fonctionnels intestinaux, d'intolérances alimentaires (allergies, conditionnements, etc.) caractérisées par une douleur viscérale chronique. Elle est particulièrement adaptée au traitement préventif ou curatif des hyperalgésies et en particulier du syndrome de l'intestin irritable (SII) quelque soit sa forme (constipation, diarrhée ou une combinaison des deux), mais également des douleurs viscérales chroniques n'entrant pas dans le cadre du SII, telles que les douleurs abdominales fonctionnelles sans trouble de l'élimination fécale (FAPS : Functional Abdominal Pain) et les douleurs liées aux intolérances alimentaires et à la maladie coeliaque. Elle est utilisable de manière préventive chez des sujets présentant des prédispositions ou une sensibilité à ce type de désordres, ou de manière curative, par exemple lors de crises ou sur des périodes plus longues. Les compositions et méthodes de l'invention permettent de réduire la souffrance des sujets, d'atténuer les symptômes ou la cause de ces désordres.

La présente invention démontre en effet de façon surprenante que la suppression de l'augmentation de la perméabilité paracellulaire associée à l'activation des récepteurs PAR (par exemple le récepteur PAR-2) empêche l'apparition de l'hyperalgie viscérale.

Un objet particulier de l'invention réside dans l'utilisation d'un inhibiteur de protéases tel que défini ci-avant pour la préparation d'un médicament destiné à contrôler, notamment à réduire, la perméabilité paracellulaire de l'épithélium intestinal chez des sujets atteints de maladies intestinales caractérisées par un état d'hyperalgésie, notamment de maladies inflammatoires chroniques caractérisées par une accumulation, dans la couche sous-muqueuse, d'immunocytes (par exemple de mastocytes et/ou de cellules entérochromaffines), par une sensibilité accrue des mécanorécepteurs pariétaux et éventuellement par une infiltration de bactéries du colon dans la couche sous-muqueuse, par exemple les hyperalgésies et notamment le syndrome de l'intestin irritable.

Un autre objet particulier de l'invention réside dans l'utilisation d'un inhibiteur de protéases tel que défini ci-avant pour la préparation d'un médicament destiné à réduire la sensibilisation aux allergènes, pathogènes et/ou molécules chimiques chez des sujets atteints ou sensibles aux maladies fonctionnelles intestinales, notamment aux affections intestinales caractérisées par une accumulation, dans la couche sous-muqueuse, d'immunocytes, notamment par exemple de mastocytes et/ou de cellules entérochromaffines, par une sensibilité accrue des mécanorécepteurs pariétaux et éventuellement par une infiltration de bactéries du colon dans la couche sous-muqueuse, par exemple les hyperalgésies et notamment le syndrome de l'intestin irritable.

Un autre objet particulier de l'invention réside dans l'utilisation d'un inhibiteur de protéases tel que défini ci-avant pour la préparation d'un médicament destiné à réduire la migration transépithéliale d'immunocytes et l'accumulation d'immunocytes dans la couche sous-muqueuse de sujets atteints d'une pathologie fonctionnelle intestinale, notamment d'une affection intestinale induisant une hyperalgésie viscérale, par exemple le syndrome de l'intestin irritable, caractérisée par une accumulation, dans la couche sous-muqueuse d'immunocytes, notamment de mastocytes et/ou de cellules entérochromaffines, par une sensibilité accrue des mécanorécepteurs pariétaux et éventuellement par une infiltration de bactéries du colon dans la couche sous-muqueuse.

L'invention est également relative à des méthodes de traitement des conditions indiquées ci-dessus, comprenant l'administration à un sujet atteint d'une pathologie intestinale ou sensible aux pathologies intestinales, d'un inhibiteur de protéases ou traitement tel que défini ci-avant. De préférence, l'inhibiteur de protéases ou le traitement est administré dans une dose efficace pour réduire la perméabilité paracellulaire de l'épithelium intestinal et/ou pour réduire la sensibilité à la douleur et/ou pour réduire la migration transépithéliale d'allergènes, de toxines, d'irritants ou de microorganismes et ainsi l'accumulation d'immunocytes dans la couche sous-muqueuse de l'intestin.

L'inhibiteur de protéases peut être administré par différentes voies et sous différentes formes. Ainsi, l'inhibiteur de protéases peut être sous forme liquide ou solide, typiquement sous forme de comprimé, gélule, capsule, ampoule ou soluté buvable, solution injectable, etc. On préfère des composés formulés sous une forme administrable par voie orale (solutés buvables, comprimés, ampoules, gélule, capsule, sirops, etc.) ou rectale (suppositoire). Le conditionnement en capsule ou gélule libérant son contenu par digestion microbienne dans le colon est particulièrement préféré, lorsque cela est possible. Bien entendu, d'autres formes d'administration sont possibles, comme des injections (intrapéritonéale, intradermique, sous-cutanée, intramusculaire, intraveineuse, intra-artérielle, etc.), des pâtes, gels, etc.

Un autre objet de l'invention réside dans une composition pharmaceutique comprenant au moins un inhibiteur de protéases et un excipient acceptable sur le plan pharmaceutique, ladite composition étant formulée préférentiellement pour une administration par voie orale ou rectale. De préférence, la composition se présente sous forme de suppositoire, ou sous forme de capsule ou gélule libérant son contenu par digestion microbienne dans le colon.

D'autres aspects et avantages de la présente invention apparaîtront à la lecture des exemples qui suivent, qui doivent être considérés comme illustratifs et non limitatifs.

### LEGENDES DES FIGURES

Figure 1 : Influence de doses croissantes d'un peptide activateur des récepteurs PAR-2 (SLIGRL) sur l'absorption d'une macromolécule (⁵¹Cr-EDTA) évaluée par le pourcentage retrouvé au niveau des urines collectées pendant 24h chez le rat.
Figure 2: Perméabilité paracellulaire colique chez la souris. Influence de différents inhibiteurs de protéases bactériennes (moyenne ± SD ; n=12) :
   Cocktail d'inhibiteurs de protéase (1/2 cachet)
   Cystéine protéase inhibiteur (100µg/ml)
   Sérine protéase inhibiteur (100 et 500 µg/ml)
   Matrice Metallo Protéase inhibiteurs (100 µg/ml)
Figure 3 : Modifications de la perméabilité paracellulaire colique engendrée par la perfusion intraluminale de trypsine et de surnageant de contenu colique chez la souris normale (témoin) et après un traitement de 10 jours par un cocktail d'antibiotiques (Néomycine : 2mk/kg/j + ampicilline : 1mg/kg/j) administré par voie orale chez la souris (moyenne ± SD ; n =12).
Figure 4 : Diminution de la sensibilité rectale à la distension par la perfusion intracolique d'un cocktail d'inhibiteurs de protéases chez le rat (moyenne ± écart-type ; n=8).
Figure 5 : Influence de la perfusion intracolique de trypsine sur la sensibilité rectale à la distension chez le rat (moyenne ± ESM ; n=8).

### EXEMPLES

### Exemple 1 : Réduction de l'hyperalgésie rectale à l'aide d'un bloqueur des jonctions serrées.

L'épithélium intestinal possède des structures de liaison des cellules épithéliales qui assurent un passage contrôlé des immunocytes dans la couche sous-muqueuse de l'intestin. Cet exemple montre que certaines molécules connues pour leur effet d'augmentation de la perméabilité paracellulaire au niveau intestinal tel que le SLIGRL favorisent l'accumulation d'immunocytes dans la sous-muqueuse intestinale (mastocytes, cellules entérochromaffines) et que cet effet peut être prévenu (e.g., inhibé, réduit) par un traitement intracolique impliquant un bloqueur des jonctions serrées.

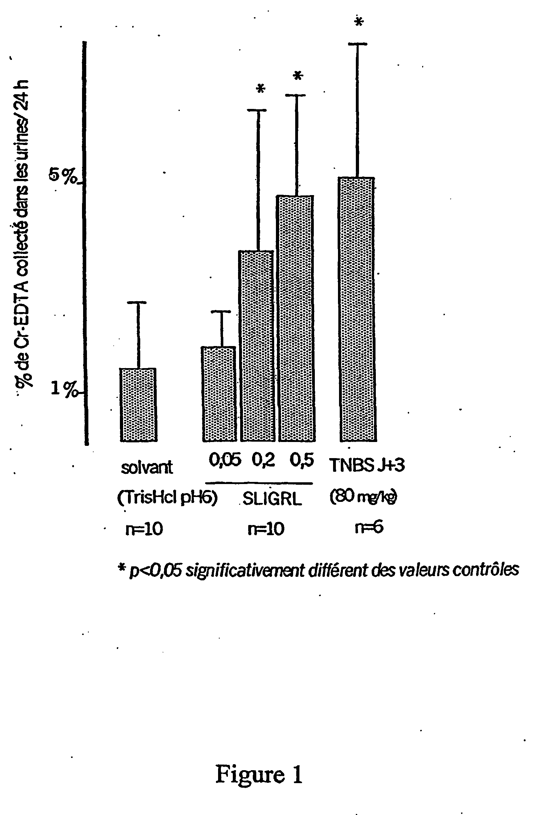

Pour réaliser cette étude, six lots de 8 rats mâles Wistar (200-250 g) ont été utilisés. Les animaux ont été munis d'un cathéter intracolique placé à demeure au niveau du côlon proximal (à 3 cm de la jonction caeco-colique). Au cours de ces essais, 4 lots de rats ont été perfusés par voie intracolique durant 10h avec une solution de ⁵¹Cr - EDTA (0.5 µc/h). Les animaux étant placés dans des cages individuelles, l'urine totale de 24h a été collectée et la mesure de sa radioactivité a permit d'évaluer la perméabilité de la muqueuse colique au ⁵¹Cr -EDTA. Des doses croissantes d'un peptide activateur du PAR-2 (SLIGRL) ont été injectées dans la lumière colique (lots 2, 3 et 4) au temps t = 0 (début de perfusion du ⁵¹ Cr - EDTA) aux doses de 0,05, 0,2 et 0,5 mg/rat, le lot 1 recevant le solvant La figure n°1 montre l'augmentation dose-dépendante de la perméabilité au ⁵¹Cr-EDTA induite par le SLIGRL montrant son effet d'augmentation de la perméabilité paracellulaire.

### Exemple 2 : Perméabilité paracellulaire colique chez la souris. Influence de différents inhibiteurs de protéases bactériennes.

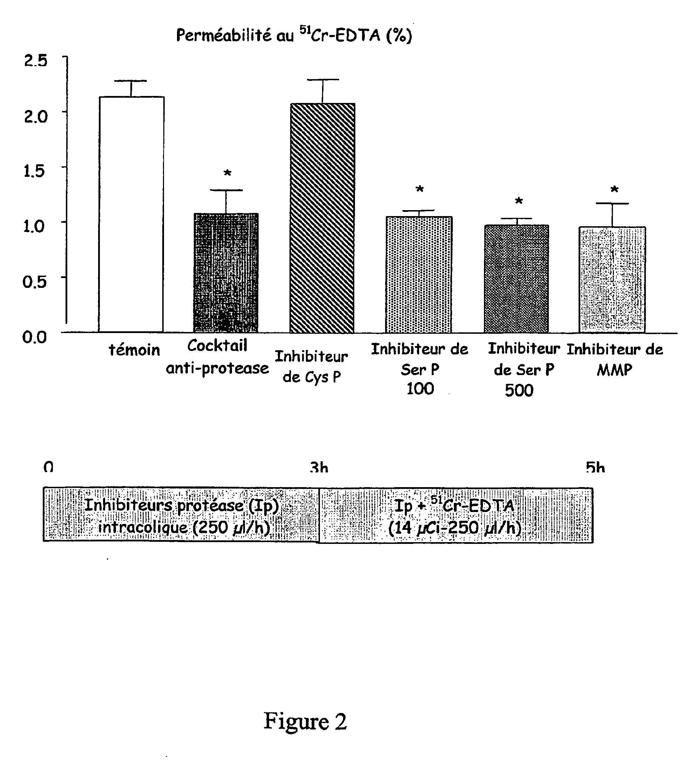

Six lots de 10 souris Swiss mâles (25-30 g) ont été utilisés pour cette étude. Les animaux ont été perfusés pendant 5 heures par voie intracolique (250 µl//h) à l'aide d'un cathéter introduit par voie rectale et fixé à la base de la queue, avec les différentes solutions d'inhibiteurs de protéases et perfusion ajoutée de ⁵¹Cr-EDTA de 3 à 5 heures (Fig.2). A t=5heures, les animaux sont abattus et après prélèvement du colon, la radioactivité totale retrouvée dans le corps témoigne de la perméabilité paracellulaire. Celle-ci apparaît significativement réduite après perfusion du cocktail d'inhibiteurs de protéases (Roche ref : 1 873 580, EDTA-free), d'un inhibiteur de sérine-protéases (Aprotinin Sigma A1153) et d'un inhibiteur non-spécifique de matricemétallo-protéases (galardin Sigma : M5939.) (Fig.2)

### Exemple 3 : Modifications de la perméabilité paracellulaire colique chez la souris engendrée par la perfusion intraluminale de trypsine et de surnageant de contenu colique et après un traitement par un cocktail d'antibiotiques.

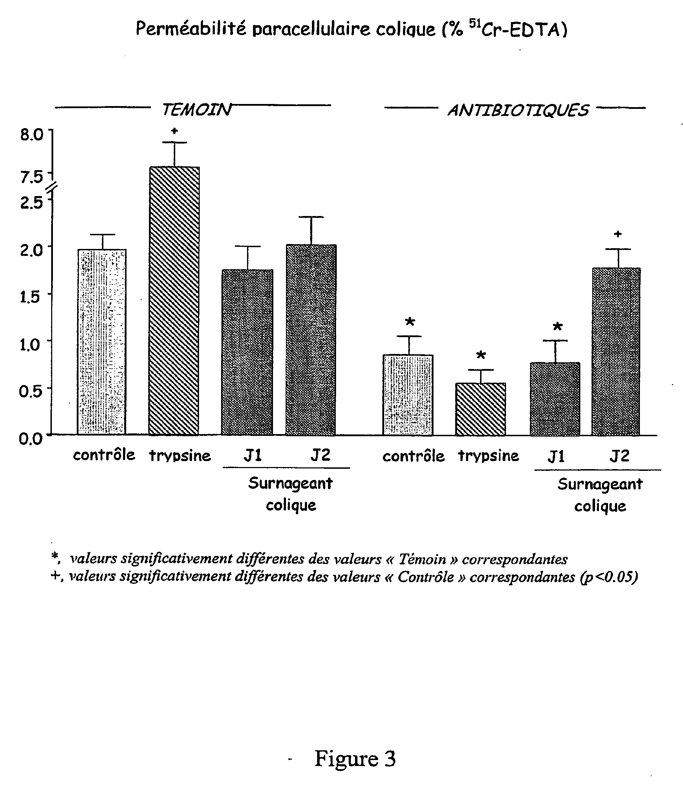

Des lots de 10 souris mâle Swiss (Janvier- France) pesant 25-30g ont été utilisés dans cette expérience. En condition basale, les effets sur la perméabilité paracellulaire colique de la perfusion intracolique de trypsine (50µl, 600U) et de surnageant de contenu colique prélevé sur des souris contrôle perfusées par voie intracolique (250µl/h) pendant 3 heures un jour (J1) ou 2 jours consécutifs (J2) ont été mesurés. Les mêmes perfusions ont ensuite été réitérées après 12 jours d'un traitement oral par un cocktail d'antibiotiques (néomycine: 2mg/kg/j+ ampicilline : 1mg/kg/j). Les résultats montrent 1) que le traitement aux antibiotiques diminue la perméabilité colique, 2) que l'augmentation de la perméabilité induite par la trypsine disparaît après traitement antibiotique induisant probablement une disparition des récepteurs épithéliaux aux protéases et 3) que chez ces animaux, la perfusion de surnageant colique augmente cette perméabilité au deuxième jour (Fig.3).

### Exemple 4: Diminution de la sensibilité rectale à la distension par la perfusion intracolique d'un cocktail d'inhibiteurs de protéases chez le rat.

Dans cette série d'essais, 3 lots de 8 rats mâles Wistar (250-300 g) ont été soumis à un protocole de distension rectale de volumes croissants (0,4 ml) de 0 à 1,6 ml réalisées à l'aide d'une sonde à embolectomie (FORGATY®). Les animaux ont été équipés au préalable d'électrodes implantées dans les muscles striés de l'abdomen permettant l'enregistrement électromyographiques des crampes abdominales reconnues comme un critère de douleur.

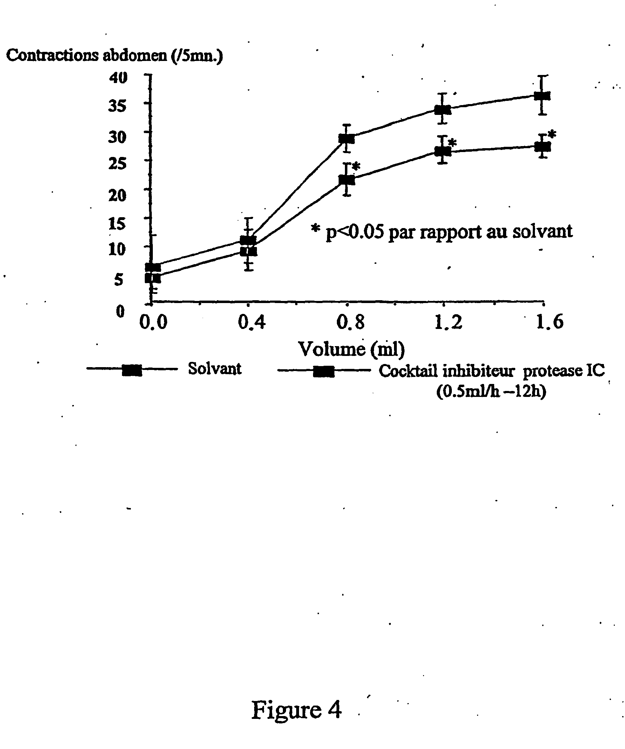

Au temps t=0 les animaux ont reçu, par voie intracolique, une perfusion pendant 12 heures du cocktail d'inhibiteurs de protéases (Roche réf. : 1 873 580) (2 cachets - 0.5ml/h) ou de son solvant NaCl 0.9%. A la fin de la perfusion, les animaux sont soumis au protocole de distension rectale. Par rapport au lot témoin, la perfusion d'inhibiteurs de protéases provoque une diminution significative de la réponse abdominale pour les volumes de distension de 0,8, 1,2 et 1,6 ml (Fig. 4) montrant ainsi que les protéases libérées par la microflore dans la lumière colique participent à la détermination de l'état basal de sensibilité rectocolique à la distension (Fig.4).

### Exemple 5 : Influence de la perfusion intracolique de trypsine sur la sensibilité rectale à la distension chez le rat

Dans cette série d'essais 2 lots de 8 rats mâles Wistar (250-300 g) ont été soumis à un protocole de distension rectale suivant des étapes croissantes (0,4 ml) de 0 à 1,6 ml réalisées à l'aide d'une sonde à embolectomie (FORGATY®). Les animaux ont été équipés au préalable d'électrodes implantées dans les muscles striés de l'abdomen permettant l'enregistrement électromyographiques des crampes abdominales reconnues comme un critère de douleur.

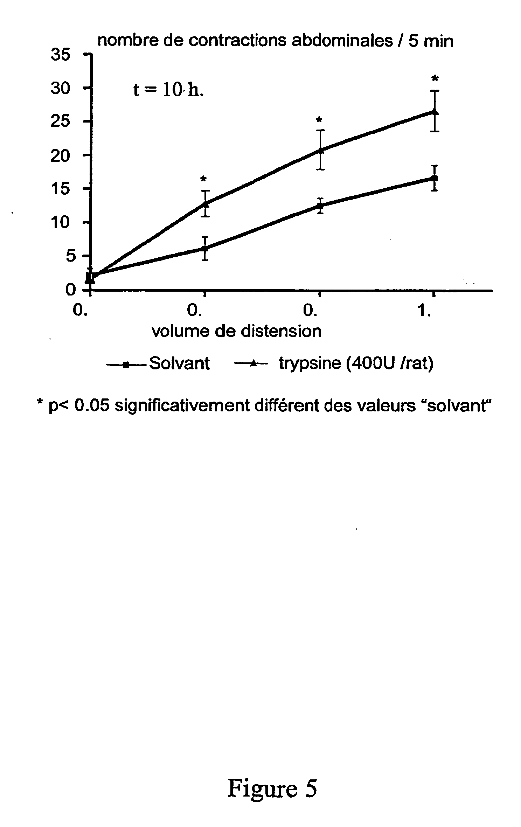

Au temps t=0, les animaux ont reçus, par voie intracolique, la trypsine (400 unités) un activateur spécifique des récepteurs PAR-2, à la dose de 20 unités (lot 1) ou son solvant (lot 2). Au temps t = 10h, après l'administration intracolique de trypsine, les animaux sont soumis au protocole de distension rectale. Par rapport au lot témoin, la trypsine provoque une augmentation significative de la réponse abdominale pour les volumes de distension de 0,8, 1,2 et 1,6 ml (Fig. 5) montrant ainsi qu'une augmentation de concentration intracolique en protéases augmente la sensibilité colique à la distension.

## Revendications

1. Utilisation d'au moins un inhibiteur de protéases, pour la préparation d'un médicament destiné au traitement préventif ou curatif des troubles fonctionnels digestifs (TFD).

2. Utilisation selon la revendication 1, **caractérisée en ce que** l'inhibiteur de protéases est un inhibiteur de protéase intracolique.

3. Utilisation selon la revendication 1 ou 2, **caractérisée en ce que** l'inhibiteur est un inhibiteur de sérine-protéases ou un inhibiteur de métalloprotéinases.

4. Utilisation selon la revendication 3, **caractérisée en ce que** l'inhibiteur de sérine-protéases est choisi parmi la serpine et ses dérivés, l'Aprotinine, la N-tosyl-L-phenylalanyl chlorométhyle cétone, la dichloroisocoumarine, la nexin-1, le AEBSF-HCl, l'Antipain, la benzamidine, la Leupeptine, le TLCK, l'Ovomucoide, le fluorure de phénylméthyle sulfonyle et les extraits de soja.

5. Utilisation selon la revendication 3, **caractérisée en ce que** l'inhibiteur de métalloprotéinases est choisi parmi l'Amastatine, l'Arphamenine, la Bestatin, la Diprotin A et le Phosphoramidon.

6. Utilisation selon la revendication 1, **caractérisée en ce que** l'inhibiteur de protéases est choisi parmi l'amprenavir, l'indinavir, le lopinavir, le ritonavir, le saquinavir, le nelfinavir et l'atazanavir.

7. Utilisation selon l'une quelconque des revendications précédentes, pour la préparation d'un médicament destiné à contrôler la perméabilité paracellulaire de l'épithelium intestinal chez des sujets atteints de maladies fonctionnelles intestinales **caractérisées par** un état d'hyperalgésie.

8. Utilisation selon la revendication 7, pour la préparation d'un médicament destiné à réduire la perméabilité paracellulaire de l'épithelium intestinal chez des sujets atteints de maladies fonctionnelles intestinales **caractérisées par** un état d'hyperalgésie.

9. Utilisation selon l'une quelconque des revendications précédentes, pour la préparation d'un médicament destiné à réduire la sensibilité à la douleur chez des sujets atteints ou sensibles aux maladies fonctionnelles intestinales **caractérisées par** un état d'hyperalgésie.

10. Utilisation selon l'une quelconque des revendications 1 à 6, pour la préparation d'un médicament destiné au traitement préventif ou curatif des troubles fonctionnels intestinaux (TFI).

11. Utilisation selon la revendication 10, **caractérisée en ce que** les troubles fonctionnels intestinaux sont choisis parmi le syndrome de l'intestin irritable (SII), les douleurs abdominales fonctionnelles sans trouble de l'élimination fécale et les douleurs liées aux intolérances alimentaires.

12. Utilisation selon l'une quelconque des revendications précédentes, **caractérisée en ce que** l'inhibiteur est administré par voie orale ou rectale.

13. Utilisation d'au moins un inhibiteur de protéases, pour la préparation d'un médicament destiné au traitement préventif ou curatif de l'hyperalgésie survenant dans le cadre de pathologies intestinales.

14. Utilisation selon la revendication 13, **caractérisée en ce que** l'inhibiteur de protéases est tel que défini à l'une des revendications 2, 3, 4, 5 et 6.

15. Produit pharmaceutique comprenant au moins un inhibiteur de protéases et au moins un autre agent actif sélectionné parmi les composés anti-cholinergiques, les prokinétiques, les anti-diarrhéiques, les laxatifs, les modificateurs de la motricité ou de la viscéro-sensibilité, en vue d'une utilisation combinée, séparée ou espacée dans le temps.

16. Produit selon la revendication 15, **caractérisé en ce que** l'inhibiteur de protéase est choisi parmi un inhibiteur de sérine-protéases et un inhibiteur de métallo-protéases.

17. Produit pharmaceutique selon l'une des revendications 15 ou 16, **caractérisé en ce qu'**il se présente sous la forme d'une capsule ou d'une gélule libérant son contenu par digestion microbienne dans le colon.

18. Inhibiteur de protéases pour une utilisation dans le traitement préventif ou curatif des troubles fonctionnels digestifs (TFD).

## Claims

1. Use of at least one protease inhibitor, for preparing a medicament for the preventive or curative treatment of functional digestive disorders (FGID).

2. Use according to claim 1, **characterized in that** the protease inhibitor is an intracolonic protease inhibitor.

3. Use according to either one of claims 1 or 2, **characterized in that** the inhibitor is an inhibitor of serine proteases or an inhibitor of metalloproteases.

4. Use according to claim 3, **characterized in that** the serine protease inhibitor is selected in the group consisting of serpin and derivatives thereof, aprotinin, N-tosyl-L-phenylalanyl chloromethyl ketone, dichloroisocoumarin, nexin-1, AEBSF-HCl, Antipain, benzamidine, leupeptin, TLCK, ovomucoid, phenylmethyl sulfonyl fluoride and soy bean extracts.

5. Use according to claim 3, **characterized in that** the metalloprotease inhibitor is selected in the group consisting of amastatin, arphamenin, bestatin, diprotin A and phosphoramidon.

6. Use according to claim 1, **characterized in that** the protease inhibitor is selected in the group consisting of amprenavir, indinavir, lopinavir, ritonavir, saquinavir, nelfinavir and atazanavir.

7. Use according to any one of the previous claims, for preparing a medicament for controlling paracellular permeability of the intestinal epithelium in subjects with functional intestinal disorders **characterized by** a state of hyperalgesia.

8. Use according to claim 7, for preparing a medicament for reducing the paracellular permeability of the intestinal epithelium in subjects with functional intestinal disorders **characterized by** a state of hyperalgesia.

9. Use according to any one of the previous claims, for preparing a medicament for reducing sensitivity to pain in subjects afflicted with or susceptible to functional intestinal disorders **characterized by** a state of hyperalgesia.

10. Use according to any one of claims 1 to 6, for preparing a medicament for the preventive or curative treatment of functional intestinal disorders (IFD).

11. Use according to claim 10, **characterized in that** the intestinal functional disorders are selected in the group consisting of irritable bowel syndrome (IBS), functional abdominal pain without defecation disorder and pain related to food intolerance.

12. Use according to any one of the previous claims, **characterized in that** the inhibitor is administered by the oral or rectal route.

13. Use of at least one protease inhibitor, for preparing a medicament for the preventive or curative treatment of hyperalgesia occurring in the context of intestinal pathologies.

14. Use according to claim 13, **characterized in that** the protease inhibitor is such as defined in any one of claims 2, 3, 4, 5 and 6.

15. Pharmaceutical product comprising at least one protease inhibitor and at least one other active agent selected in the group consisting of anticholinergic compounds, prokinetics, antidiarrheals, laxatives, modifiers of motility or visceral sensitivity, in view of a use that is combined, separate or spread out over time.

16. Product according to claim 15, **characterized in that** the protease inhibitor is selected in the group consisting of an inhibitor of serine proteases and an inhibitor of metalloproteases.

17. Pharmaceutical product according to either one of claims 15 or 16, **characterized in that** said product is formulated as a capsule or a gelatin capsule which releases its contents by microbial digestion in the colon.

18. Protease inhibitor for use in the preventive or curative treatment of functional digestive disorders (FGID).

## Patentansprüche

1. Verwendung wenigstens eines Proteasenhemmers zur Herstellung eines Medikaments für die präventive oder kurative Behandlung von funktionellen Verdauungsstörungen.

2. Verwendung gemäß Anspruch 1, **dadurch gekennzeichnet, dass** der Proteasenhemmer ein Hemmer einer intrakolonischen Protease ist.

3. Verwendung gemäß Anspruch 1 oder 2, **dadurch gekennzeichnet, dass** der Hemmer ein Serinproteasenhemmer oder ein Metalloproteasenhemmer ist.

4. Verwendung gemäß Anspruch 3, **dadurch gekennzeichnet, dass** der Serinproteasenhemmer aus Serpin und seinen Derivaten, Aprotinin, N-Tosyl-L-phenylalanylchlormethylketon, Dichlorisocumarin, Nexin-1, AEBSF-HCl, Antipain, Benzamidin, Leupeptin, TLCK, Ovomucoid, Phenylmethylsulfonylfluorid und Sojaextrakten ausgewählt ist.

5. Verwendung gemäß Anspruch 3, **dadurch gekennzeichnet, dass** der Metalloproteasenhemmer aus Amastatin, Arphamenin, Bestatin, Diprotin A und Phosphoramidon ausgewählt ist.

6. Verwendung gemäß Anspruch 1, **dadurch gekennzeichnet, dass** der Proteasenhemmer aus Amprenavir, Indinavir, Lopinavir, Ritonavir, Saquinavir, Nelfinavir und Atazanavir ausgewählt ist.

7. Verwendung gemäß einem der vorhergehenden Ansprüche zur Herstellung eines Medikaments für die Kontrolle der parazellulären Permeabilität des Darmepithels bei Personen, die an funktionellen Darmkrankheiten leiden, die durch einen Zustand der Hyperalgesie **gekennzeichnet** sind.

8. Verwendung gemäß Anspruch 7 zur Herstellung eines Medikaments für die Verringerung der parazellulären Permeabilität des Darmepithels bei Personen, die an funktionellen Darmkrankheiten leiden, die durch einen Zustand der Hyperalgesie **gekennzeichnet** sind.

9. Verwendung gemäß einem der vorhergehenden Ansprüche zur Herstellung eines Medikaments für die Verringerung der Schmerzempfindlichkeit bei Personen, die an funktionellen Darmkrankheiten leiden oder dafür empfänglich sind, die durch einen Zustand der Hyperalgesie **gekennzeichnet** sind.

10. Verwendung gemäß einem der Ansprüche 1 bis 6 zur Herstellung eines Medikamentes für die präventive oder kurative Behandlung von funktionellen Darmstörungen.

11. Verwendung gemäß Anspruch 10, **dadurch gekennzeichnet, dass** die funktionellen Darmstörungen aus Reizdarmsyndrom (RDS), funktionellen Abdominalschmerzen ohne Störung der Defäkation und Schmerzen, die mit Nahrungsmittelunverträglichkeiten verbunden sind, ausgewählt sind.

12. Verwendung gemäß einem der vorhergehenden Ansprüche, **dadurch gekennzeichnet, dass** der Hemmer über den oralen oder rektalen Weg verabreicht wird.

13. Verwendung wenigstens eines Proteasenhemmers zur Herstellung einer Medikaments für die präventive oder kurative Behandlung der Hyperalgesie, die im Rahmen von Darmkrankheiten auftritt.

14. Verwendung gemäß Anspruch 13, **dadurch gekennzeichnet, dass** der Proteasenhemmer wie in einem der Ansprüche 2, 3, 4, 5 und 6 definiert ist.

15. Pharmazeutisches Produkt, umfassend wenigstens einen Proteasenhemmer und wenigstens einen weiteren Wirkstoff, der aus anticholinergen Verbindungen, Prokinetika, Antidiarrhoika, Laxativa, Modifikatoren der Motorik oder der Viszerosensibilität ausgewählt ist, im Hinblick auf eine kombinierte, getrennte oder in zeitlichen Abständen erfolgende Verwendung.

16. Produkt gemäß Anspruch 15, **dadurch gekennzeichnet, dass** der Proteasenhemmer aus einem Serinproteasenhemmer oder einem Metalloproteasenhemmer ausgewählt ist.

17. Pharmazeutisches Produkt gemäß einem der Ansprüche 15 oder 16, **dadurch gekennzeichnet, dass** es in Form einer Kapsel oder Gelatinekapsel vorliegt, die ihren Inhalt aufgrund mikrobiellen Abbaus im Colon freisetzt.

18. Proteasenhemmer zur Verwendung in der präventive oder kurative Behandlung von funktionellen Verdauungsstörungen.
